# EUROPEAN PATENT APPLICATION

(11) **EP 3 144 305 A1**
(43) Date of publication of application: **22.03.2017**
(21) Application number: 15793046.2
(22) Date of filing: 11.05.2015
(51) Int. Cl.: C07D 401/06, C07B 53/00, C07B 57/00, C07D 213/50, C07D 213/64, C07D 213/84, C07D 213/89

(54) **METHOD FOR PRODUCING 2-PYRIDONE COMPOUND**

(30) Priority: 13.05.2014 JP 2014099755
(71) Applicant: Nissan Chemical Industries, Ltd., Chiyoda-ku Tokyo 101-0054 (JP)
(72) Inventor: YOSHINO, Hironobu, Funabashi-shi Chiba 274-8507 (JP); UMEDA, Yasuhiro, Funabashi-shi Chiba 274-8507 (JP); TAKEOKA, Jun, Funabashi-shi Chiba 274-8507 (JP); NAGAYA, Akihiro, Funabashi-shi Chiba 274-8507 (JP); SUGAWARA, Yudai, Funabashi-shi Chiba 274-8507 (JP); YOSHINO, Madoka, Funabashi-shi Chiba 274-8507 (JP)
(74) Representative: Hartz, Nikolai
(86) International application number: PCT/JP2015/063509
(87) International publication number: WO 2015/174377

(57) **Abstract**

To provide a method for producing a compound represented by the formula (1) which is a 2-pyridone compound useful as a pharmaceutical or an intermediate for a pharmaceutical, etc. at a high yield.

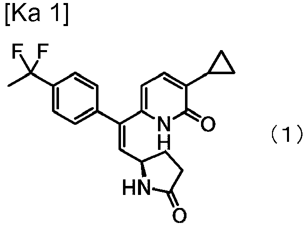

A method for producing a 2-pyridone compound represented by the formula (1), which comprises reacting a 6-benzoyl-2-pyridone compound represented by the formula (3) with a sulfone compound represented by the formula (4):

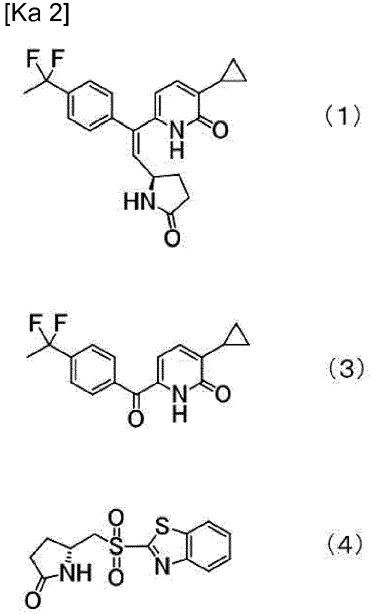

## Description

### TECHNICAL FIELD

The present invention relates to a method for producing a 2-pyridone compound.

### BACKGROUND ART

The 2-pyridone compound (compound (2)) represented by the formula (2) is a compound included in the claims in a compound patent (Patent Document 1) for a therapeutic agent for diabetes, which claims a series of 2-pyridone compounds, and the possibility for its use as pharmaceuticals has been known:

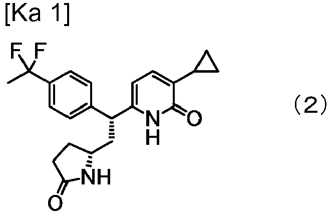

Accordingly, a specific method for producing the compound (2) has been desired.

### PRIOR ART DOCUMENT

### PATENT DOCUMENT

Patent Document 1: WO2011/068211

### DISCLOSURE OF INVENTION

### TECHNICAL PROBLEM

An object of the present invention is to provide a method for producing a 2-pyridone compound.

### SOLUTION TO PROBLEM

As a result of extensive studies to accomplish the above object, the present inventors have established a synthetic route for a 6-benzoyl-2-pyridone compound which is a key intermediate and found that a compound (1) can be obtained by using the intermediate as a starting material.

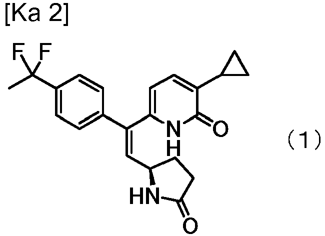

In the production of the compound (1) from the 6-benzoyl-2-pyridone compound as the starting material, the present inventors have tried applying the general synthetic method described in Patent Document 1. As a result, they have found that in addition to the desired compound (1) in the form of Z-isomer, a compound in the form of E-isomer is formed as a by-product. The yield of the desired compound (1) deteriorates due to the by-production of the compound in the form of E-isomer, and it is necessary to purify the desired compound (1) by column chromatography. Thus, the method described in Patent Document 1 is problematic as an industrial production method. Accordingly, the present inventors have conducted extensive studies for the production of the compound (1) and have found the method for selectively producing the compound (1) in the form of Z-isomer.

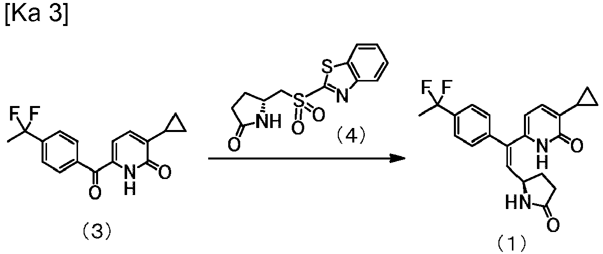

Further, the present inventors have found for the first time that the compound (1) obtained by the above production method is crystallized in the form of a sodium salt and thus have developed a purification method which does not require column chromatography.

Further, the present inventors have found a production method to be industrially applied in which the compound (2) is formed from the compound (1) in the following scheme. Thus, the present inventors have accomplished the present invention.

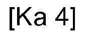

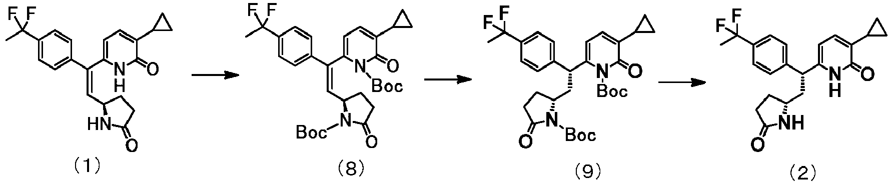

That is, the present invention has the following features.
(I) A method for producing a 2-pyridone compound represented by the formula (1), which comprises reacting a 6-benzoyl-2-pyridone compound represented by the formula (3) with a sulfone compound represented by the formula (4): 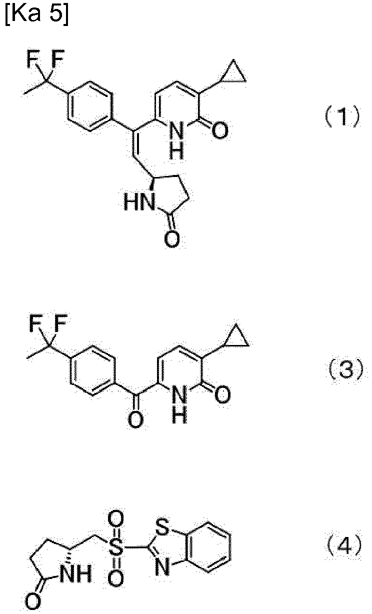
(II) The production method according to (I), wherein the reaction is carried out in the presence of a urea derivative.
(III) The production method according to (II), wherein the urea derivative is 1,3-dimethyl-2-imidazolidinone.
(IV) A compound represented by the formula (1): 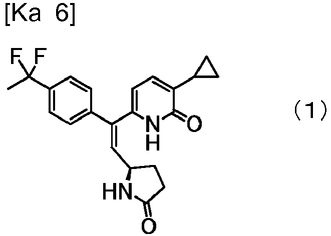
(V) A compound represented by the formula (3): 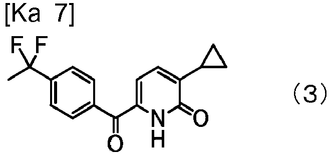
(VI) A sodium salt of a compound represented by the formula (1): 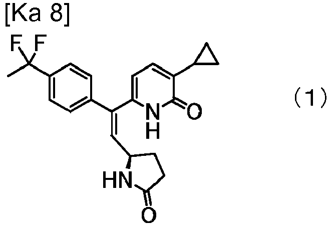
(VII) A compound represented by the formula (6): 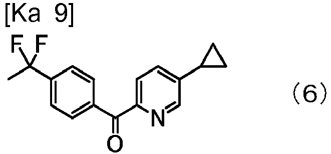
(VIII) A compound represented by the formula (7): 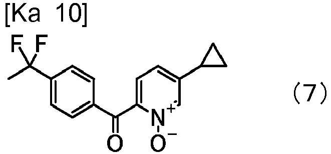
(IX) A compound represented by the formula (8): 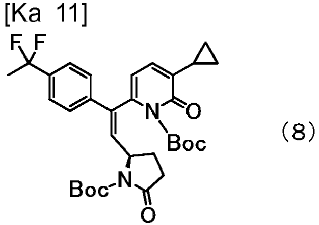
(X) A compound represented by the formula (9): 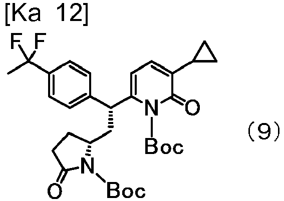
(XI) A compound represented by the formula (10):

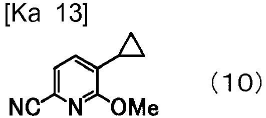

### ADVANTAGEOUS EFFECTS OF INVENTION

According to the present invention, it has been made possible to provide a method for producing a 2-pyridone compound which is useful as a pharmaceutical or its intermediate.

### BRIEF DESCRIPTION OF DRAWING

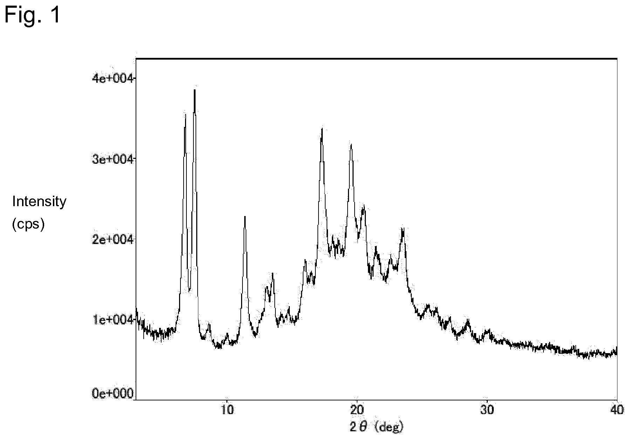

Fig. 1 shows a powder X-ray diffraction pattern of a crystal of the sodium (R, Z)-3-cyclopropyl-6-{1-[4-(1,1-difluoroethyl)phenyl]-2-[5-oxopyrrolidin-2-yl]vinyl}pyridin-2-olate of the present invention.

### DESCRIPTION OF EMBODIMENTS

Now, the present invention will be described in detail.

In this invention, "n" means normal, "i" means iso, "s" and "sec" mean secondary, "t" and "tert" mean tertiary, "c" means cyclo, "o" means ortho, "m" means meta, "p" means para, "Boc" means t-butoxycarbonyl, "Me" means methyl. Further, "(E)" means E-isomer, and "(Z)" means Z-isomer.

A halogen atom means a fluorine atom, a chlorine atom, a bromine atom or an iodine atom.

A C₁₋₄ alkyl group means a linear or branched alkyl group having from 1 to 4 carbon atoms, such as a methyl group, an ethyl group, an n-propyl group, an i-propyl group, an n-butyl group, an i-butyl group, an s-butyl group or a t-butyl group.

Now, each production route of the present invention will be described in detail.

### 1. Method for producing the compound (3)

General processes for producing the compound (3) will be illustrated as schemes 1 to 3, however, they are examples of general production processes, and the production process is by no means restricted thereto. The compound (3) can also be produced by methods well known to those skilled in the art such that the order to carry out steps is changed, a protecting group is added to a hydroxy group or the like, a reaction is carried out, and then the protecting group is released in a post step, an additional step is added in each step, etc.

In the production of the compound of the present invention, the method for protecting or deprotecting functional groups contained in starting materials, intermediates, etc. may be carried out in accordance with methods well known to those skilled in the art, for example, the method described in Greene's Protective Groups in Organic Synthesis, published by John Wily and Sons, year 2006, etc.

### Scheme 1: Method for producing the compound (3) from the compound (1-a)

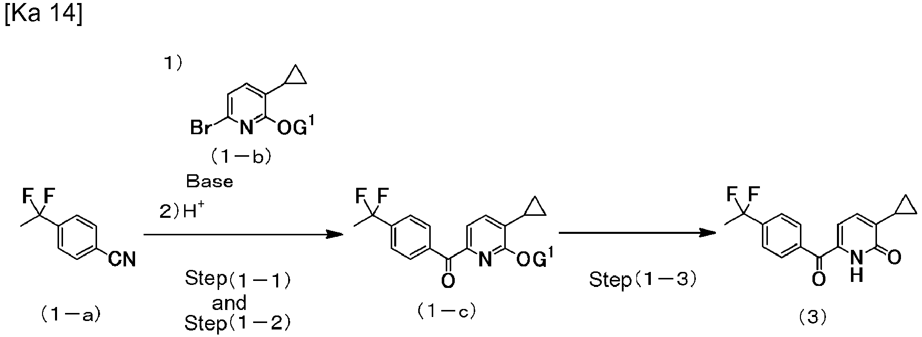

In Scheme 1, G¹ is a protecting group of hydroxy group in the hydroxy pyridyl group.

The compound (1-a) and the compound (1-b) can be obtained by the methods described in WO2008/103185 or methods based on it.

### Step (1-1) and Step (1-2):

"Addition reaction" using the compound (1-a) and an anion such as a lithium reagent e.g. heteroaryl lithium or a Grignard reagent e.g. heteroaryl magnesium bromide, is carried out, and the obtained compound is treated with an acid such as hydrochloric acid to obtain the compound (1-c).

The "addition reaction" may, for example, be a method of generating an anion by using the compound (1-b) as a substrate and an organic metal reagent such as n-butyl lithium, sec-butyl lithium, tert-butyl lithium or diisopropyl magnesium bromide, a metal reagent such as magnesium or a base such as lithium bis(trimethylsilyl)amide or potassium bis(trimethylsilyl)amide in an inert solvent at a temperature of from -78°C to 200°C, followed by reacting the anion with a nitrile compound of the compound (1-a). Step (1-3):
The compound (3) can be produced by carrying out "deprotection reaction" of a protecting group G¹ in the compound (1-c).

The "deprotecting reaction" may, for example, be a deprotecting reaction such as (i) in a case where the protecting group G¹ is an alkyl group or an allyl group, a method in which the protecting group G¹ is removed by a hydrolytic reaction in the presence of an acid or a strong acid in an inert solvent at a temperature of from 0°C to 200°C, a method using trimethylsilyl iodide or the like or a method using aluminum chloride and an alkylthiol. Further, (ii) in a case where the protecting group G¹ is a benzyl group, a 4-methoxybenzyl group, a 2,4-dimethoxybenzyl group, a benzyloxycarbonyl group, a benzhydryl(diphenylmethyl) group or the like, the deprotecting reaction may, for example, be a method in which the protecting group G¹ is removed by a hydrogenolysis reaction using an catalytic amount of palladium-activated carbon, rhodium-activated carbon or the like in the presence of or the absence of an acid in an inert solvent at a temperature of from 0°C to 80°C or a method using an oxidizing agent such as ammonium cerium(IV) nitrate or 2,3-dichloro-5,6-dicyano-p-benzoquinone. Further, in a case where the protecting group G¹ is an alkyl group, G¹ represents a C₁₋₄ alkyl group, preferably a methyl group or an ethyl group, more preferably a methyl group.

### Scheme 2: Method for producing the compound (3) from the compound (2-a)

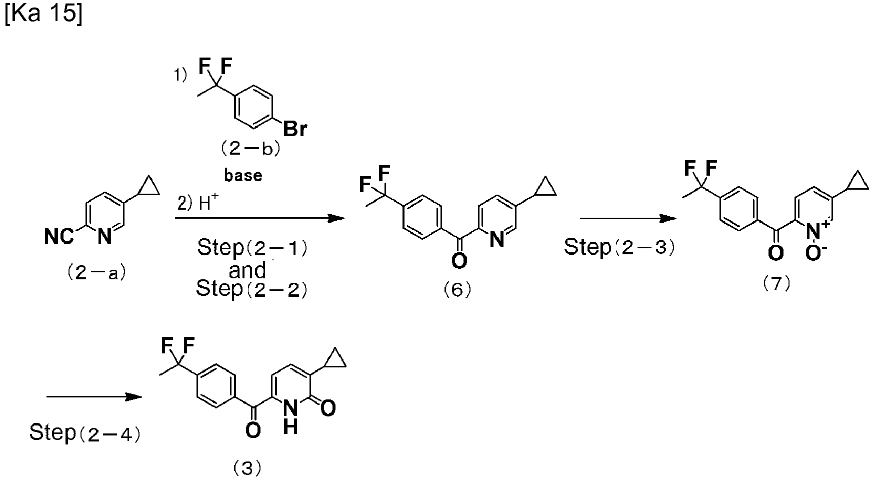

The compound (2-a) and the compound (2-b) are available as commercial compounds.

### Step (2-1) and Step (2-2):

"Addition reaction" using the compound (2-a) and an anion such as a lithium reagent e.g. phenylaryl lithium or Grignard reagent e.g. phenylaryl magnesium bromide is carried out, and the obtained compound is treated with an acid such as hydrochloric acid to produce the compound (6).

As the "addition reaction", the substantially same reaction as the addition reaction in the step (1-1) and the step (1-2) may be mentioned.

### Step (2-3):

The compound (7) can be produced by subjecting the compound (6) to "oxidation reaction" with an oxidizing agent.

The "oxidation reaction" may, for example, be a method in which the compound (6) is reacted with an "oxidizing agent" in an inert solvent such as chloroform or water at -20°C to 60°C to obtain the compound (7).

The "oxidizing agent" may, for example, be a peracid such as metachloroperoxybenzoic acid. The peracid may also be generated by combining hydrogen peroxide and an acid or an acid anhydride in the system and used. Step (2-4):
The compound (3) can be produced by subjecting the compound (7) to "transferring reaction" with an acid anhydride.

The "transferring reaction" may, for example, be a method in which the compound (7) is reacted with an acid anhydride such as trifluoroacetic acid anhydride in an inert solvent such as chloroform, tetrahydrofuran, 2-methyltetrahydrofuran or methyl t-butyl ether at a temperature of from -20°C to 60°C to obtain the compound (3).

### Scheme 3: Method for producing the compound (3) from the compound (3-a)

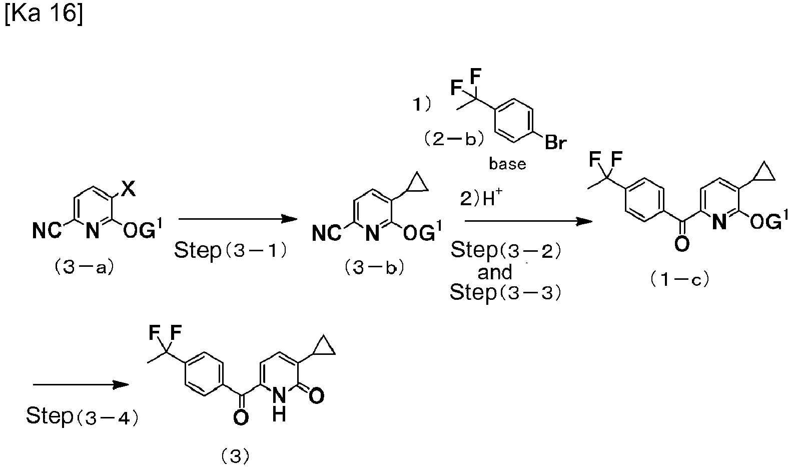

In the scheme 3, X is a halogen atom, and G¹ is the same as defined above.

The compound (3-a) may be obtained by the method described in WO2008/103185 or a method based on it.

### Step (3-1):

The compound (3-b) can be produced by subjecting the compound (3-a) as a substrate to "coupling reaction" with a cyclopropyl magnesium compound, a cyclopropyl zinc compound or cyclopropyl boronic acid.

The "coupling reaction" may, for example, be a method of a reaction with a cyclopropyl magnesium compound, a cyclopropyl zinc compound or a cyclopropyl boronic acid in the presence of a palladium, nickel or iron catalyst in an inert solvent such as 1,2-dimethoxyethane, methylene chloride, acetonitrile, toluene, tetrahydrofuran, 2-methyltetrahydrofuran, N-methylpyrrolidone or 1,4-dioxane at -20°C to 40°C.

The palladium catalyst used in the "coupling reaction" may, for example, be a palladium catalyst well known for those skilled in the art, such as tetrakistriphenylphosphine palladium(0), bis(dibenzylideneacetone) palladium(0), bis(triphenylphosphine) palladium(II) dichloride, bis(triphenylphosphine) palladium(II) acetate or [1,1'-bis(diphenylphosphine)ferocene] palladium(II) dichloride-dichloromethane complex (1:1). Further, a palladium(0) catalyst can be formed in the system by using palladium acetate (II) or palladium-activated carbon and triphenylphosphine and used for the reaction.

The nickel catalyst used for the "coupling reaction" may, for example, be a nickel catalyst well known for those skilled in the art, such as bis(triphenylphosphine) nickel(II) dichloride. Further, a nickel catalyst may be formed in the system by using nickel chloride(II) and triphenylphosphine and used for the reaction.

The iron catalyst used for the "coupling reaction" may, for example, be an iron catalyst well known for those skilled in the art, such as tris(2,4-pentanedionate) iron(III). Further, an iron catalyst may be formed in the system and used for the reaction.

### Step (3-2) and Step (3-3):

The compound (1-c) can be produced by carrying out "addition reaction" using a compound (3-b) and an anion such a lithium reagent e.g. phenylaryl lithium or a Grignard reagent e.g. phenylaryl magnesium bromide, followed by treating the obtained compound with an acid such as hydrochloric acid.

As the "addition reaction", the addition reactions described in step (1-1) and step (1-2) in the above scheme 1 may be mentioned.

### Step (3-4):

The compound (3) can be produced by carrying out the reaction of step (1-3) in the above scheme 1.

### 2. Method for producing the compound (1)

The method for producing the compound (1) is illustrated in scheme 4.

### Scheme 4: Method for producing the compound (1) from the compound (3)

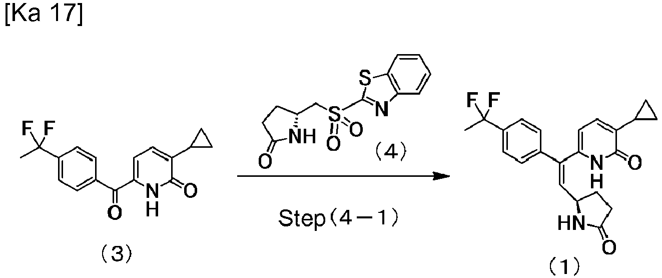

### Step (4-1):

The compound (1) can be produced by subjecting the compound (3) as a substrate to "coupling reaction" with the compound (4) in the presence of a base.

The compound (4) used for the "coupling reaction" can be obtained by the method described in WO2008/103185.

The compound (1) to be obtained by the "coupling reaction" is obtained as a mixture containing E-isomer.

The base to be used for the "coupling reaction" is not particularly restricted, and a base may be solely used, or a mixture containing plural bases may be used. The base is preferably an organic base or an organic metal base, more preferably an alkali metal base of an amine in which silyl groups are substituted by alkyls, allyls or both of them, further preferably lithium bis(trimethylsilyl)amide.

The base is preferably used in a molar equivalent amount of from 1.0 to 20.0, more preferably in a molar equivalent amount of from 3.0 to 10.0 to the compound (3).

The compound (4) is preferably used in a molar equivalent amount of from 1.0 to 10.0, more preferably in a molar equivalent amount of from 1.0 to 3.0 to the compound (3).

The "coupling reaction" is preferably carried out in the presence of a solvent, and the solvent to be used is not particularly restricted, so far as the reaction is not impaired. As preferred examples of the solvent, an aliphatic hydrocarbon (such as hexane or heptane), an aromatic hydrocarbon (such as benzene, toluene or xylene), an ether (such as diethyl ether, diisopropyl ether, tetrahydrofuran, 1,4-dioxane or t-butyl methyl ether), a halogenated aliphatic hydrocarbon (such as methylene chloride, chloroform or dichloroethane), a nitrile (such as acetonitrile or propionitrile) or an amide (N,N-dimethylformamide or N,N-dimethylacetoamide) may be mentioned. The solvent is preferably an aliphatic hydrocarbon or an ether, more preferably hexane or tetrahydrofuran, particularly preferably tetrahydrofuran.

The solvent may be used alone, or plural solvents may be used in combination. Further, the amount of the solvent to be used is optionally adjusted depending on a type of a substrate, since whether the substrate is crystal or not, whether the viscosity is high or not, etc. influence in general. The amount of the solvent to be used may be a range where a part of the substrate is dissolved, however, from the viewpoint of the influence of the stirring efficiency and the volume efficiency, etc., the amount of the solvent to be used is usually from 1 to 50 wt%, preferably from 2 to 20 wt%, more preferably from 3 to 10 wt%, as the substrate concentration of the compound (3).

The "coupling reaction" may be carried out at any of from -78°C to the boiling point of a reaction medium, however, from the handling of the reaction and the industrial viewpoint, the coupling reaction is usually carried out at -40°C to 60°C, preferably -30°C to 50°C, more preferably -20°C to 40°C.

The "coupling reaction" may be carried out in the presence of an additive. The additive is preferably a urea derivative (such as 1,3-dimethyl-2-imidazolidinone, 1,3-dimethyl-3,4,5,6-tetrahydro-2(1H)-pyrimidinone or tetramethylurea), more preferably 1,3-dimethyl-2-imidazolidinone. When the coupling reaction is carried out in the presence of the urea derivative, the proportion of E-isomer and Z-isomer of a compound to be formed changes, and the selectivity and the yield of the compound (1) which is Z-isomer improve.

In a case where the additive is used, the additive may be solely used, or plural additives may be used in combination. The amount of the additive to be used may be optionally adjusted depending on the type of the substrate, and the amount of the additive to be used is from 0.1 to 100 times by weight, preferably from 1 to 20 times by weight, more preferably from 2 to 6 times by weight, per the compound (3) as the substrate.

After the termination of the "coupling reaction", an acidic aqueous solution such as sulfuric acid aqueous solution is added to a reaction solution, and the reaction solution is stirred, whereby an organic metal compound is decomposed, and liquid-liquid extraction is carried out to remove mainly components derived from bases. Then, an alkaline solution such as a sodium carbonate aqueous solution is added, and liquid-liquid extraction is carried out to extract the desired product. The obtained organic layer is purified by column chromatography, crystallization or the like to obtain the desired product.

### 3. Method for producing a sodium salt of a compound represented by the formula (1)

Scheme 5 shows a method for producing a sodium salt (hereinafter referred to also as "compound (5)") of a compound represented by the formula (1).

### Scheme 5: Method for producing the compound (5) from the compound (1)

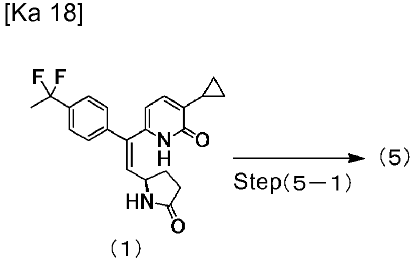

### Step (5-1):

The compound (1) as the substrate can be formed into a sodium salt with a sodium alkoxide to produce the compound (5). By crystallizing the compound (5), the compound (5) having a high purity can be formed. A solvent for salt formation or crystallization is preferably an alcohol solvent or an ester solvent. The sodium alkoxide is preferably dissolved in an alcohol solvent for use, and the compound (1) is preferably dissolved in an ester solvent for use.

The sodium alkoxide to be used is a C₁₋₄ alkoxide such as sodium methoxide, sodium ethoxide, sodium n-propoxide, sodium i-propoxide, sodium n-butopoxide, sodium s-butopoxide or sodium t-butopoxide, preferably sodium methoxide.

The sodium alkoxide to be used is more preferably a solution of an alcohol corresponding to an alkoxide.

Such a sodium alkoxide may be used as a mixture with other sodium alkoxide in an optional proportion.

Further, such a sodium alkoxide is preferably used in a molar equivalent amount of from 1.0 to 10.0, more preferably in a molar equivalent amount of from 1.0 to 3.0, per the compound (1).

The alcohol solvent to be used is a C₁₋₄ alcohol such as methanol, ethanol, n-propanol, i-propanol, n-butanol, s-butanol or t-butanol, preferably methanol. An alcohol may be added as a solution of the sodium alkoxide.

Such a solvent may be mixed with another solvent in an optional proportion for use.

The alcohol solvent may be solely used, or plural solvents may be mixed for use. Further, the amount of the solvent to be used may be optionally adjusted depending on the type of the sodium alkoxide, since in a case where the sodium alkoxide is dissolved, the solubility also influences. The amount of the solvent to be used may be a ranged where a part of the sodium alkoxide can be dissolved, however, the amount of the solvent to be used is usually from 1 to 90 wt%, preferably from 5 to 60 wt%, more preferably from 10 to 40 wt%, as the concentration of the sodium alkoxide.

The ester solvent to be used is an ester of formic acid (methyl formate, ethyl formate or n-propyl formate) or an ester of acetic acid (methyl acetate, ethyl acetate, n-propryl acetate, i-propyl acetate, n-butyl acetate, i-butyl acetate or t-butyl acetate), preferably ethyl acetate.

Such a solvent may be mixed with another solvent in an optional proportion for use.

The ester solvent may be solely used, or plural solvents may be mixed for use. Further, the amount of the solvent to be used may be optionally adjusted depending on the type of the substrate, since whether the substrate is crystal or not, whether the viscosity is high or not, etc. influence in general. The amount of the solvent to be used may be a ranged where a part of the substrate is dissolved, however, from the viewpoint of the stirring efficiency, the influence of the volume efficiency, etc., the amount of the solvent to be used is usually from 1 to 50 wt%, preferably from 2 to 20 wt%, more preferably from 3 to 10 wt%, as the substrate concentration of the compound (1).

The compound (1) is crystallized by any one method of mixing with a sodium alkoxide, mixing with a sodium alkoxide, followed by heating, cooling, concentrating or dissolving followed by adding a solvent (poor solvent) having a low solubility, or crystallized by a method combining them.

Unless otherwise specified, the temperature of the crystallization is from -20°C to 80°C, preferably from -10°C to 50°C.

A seed crystal may be used for the crystallization. The seed crystal may be obtained by a method known for those skilled in the art, such as scratching a wall of a container in which a solution of the desired product is added by a spatula.

The compound (5) which is a sodium salt of the compound (1) is a salt formed from the compound (1) and a sodium alkoxide and is a salt comprising an anion of the compound (1) and a sodium cation. Further, the proportion of components of the salt is anion of the compound (1) : sodium cation = 1:1.

Further, the structure of the compound (5) may be a compound represented by the formula (5A), the formula (5B) or the formula (5C). In the present invention, the compound (5) means one type of the compounds represented by the formula (5A), the formula (5B) and the formula (5C) or a mixture of two or more of them.

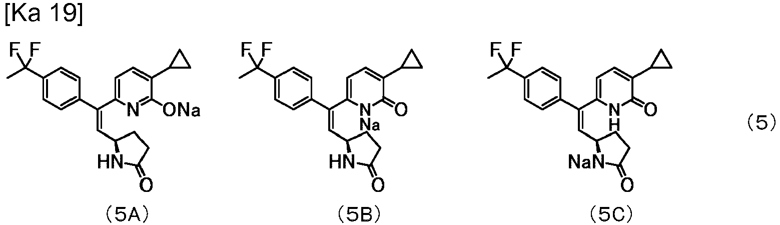

The structure of the crystal can be analyzed by powder X-ray diffraction measurement. The position of a peak (peak value) obtained by the powder X-ray diffraction measurement is represented by 2θ. The peak value may vary depending on measurement condition or the like in some cases. Further, the same or the difference of the crystal form should be determined by comprehensively analyzing measurement condition, a peak value, a diffraction pattern, etc.

The error of the powder X-ray diffraction peak is usually ±0.2, and taking the error of the crystal described in Example 6 into the consideration, the peak value is usually 2θ = 6.9 ± 0.2, 7.6 ± 0.2, 8.8 ± 0.2, 11.5 ± 0.2, 13.1 ± 0.2, 13.6 ± 0.2, 16.1 ± 0.2, 17.4 ± 0.2, 19.6 ± 0.2, 20.7 ± 0.2 or 23.6 ± 0.2.

### 4. Method for producing the compound (2)

The compound (2) can be derived from the compound (5) and the compound (1). The general production method is shown in scheme 6, however, scheme 6 is an example of a general production method, and the production method is by no means restricted thereto. The compound (2) can be also produced by a method well known for those skilled in the art, for example, changing the order of steps to be carried out, adding a protective group to an amide group or the like followed by carrying out reaction and deprotecting in a subsequent step, or adding a new step between the respective steps.

In the synthesis of the compound (2), the method for appropriately protection or deprotecting functional groups contained the starting materials, the intermediates, etc. can be carried out in accordance with a method well known for those skilled in the art similarly to the general production method of the compound (3).

### Scheme 6: Method for producing the compound (2) from the compound (5)

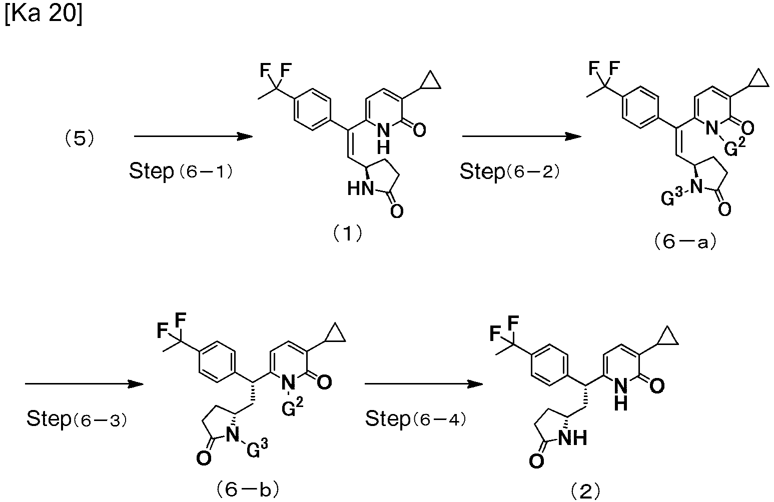

In Scheme 6, G² is a protective group for the nitrogen atom in the 2-pyridone group. G³ is a protective group for the nitrogen atom in the pyrrolidinyl group substituted by an oxo group.

### Step (6-1):

The compound (1) can be obtained by subjecting the compound (5) to liquid-liquid extrqaction from an aqueous solution such as an acid or a salt in an organic solvent to be separated from water.

### Step (6-2):

The compound (1) is reacted with di-tert-butyl dicarbonate or the like to produce a compound (6-a) having a protective group G² and a protective group G³.

### Step (6-3):

The compound (6-b) can be produced by reducing the compound (6-a) as a substrate by "catalytic hydrogenation reaction" with a catalytic amount of palladium-activated carbon, rhodium-activated carbon, platinum-activated carbon or the like in an inert solvent at -20°C to 80°C. In this production, as a case requires, an acid or a base may be added.

### Step (6-4):

The compound (2) can be produced by carrying out "deprotection reaction" of protective groups G² and G³ in the compound (6-b).

The "deprotection reaction" may be a method using an acid such as hydrochloric acid or trifluoroacetic acid.

### EXAMPLES

Now, the present invention will be described in detail with reference to Examples, however, the present invention is by no means restricted thereto.

In the silica gel column chromatography, "silica gel 60" manufactured by KANTO CHEMICAL CO., INC., "PSQ60B" manufactured by FUJI SILYSIA CHEMICAL LTD., or a packed column (YAMAZEN Hi-Flash™ Column, MORITEX Purif Pack or Biotage (registered trademark) SNAP KP-Sil Catridge) was used.

Abbreviations used in the present specification have the following means.
- s:: singlet
- d:: doublet
- t:: triplet
- q:: quartet
- dd:: double doublet
- m:: multiplet
- br:: broad
- J:: coupling constant
- Hz:: Hertz
- CDCl₃:: deuterated chloroform
- V/V:: volume/volume

In a case where ¹H-NMR data are mentioned, the data represent chemical shift δ (unit: ppm) (split pattern, integrated value) of signals wherein tetramethylsilane was used as an internal standard substance. ¹H-NMR (proton magnetic resonance spectrum) was measured by the following Fourier transform type NMR.
300 MHz: JNM-ECP300 (JEOL), JNM-ECX300 (JEOL)
600 MHz: JNM-ECA600 (JEOL)
ACD/SpecManager ver. 12.01 (trade name) was used for analysis.
MS (mass spectrum) was measured by the following devices.
micromass ZQ (Waters)
LTQ XL (Thermo Fisher Scientific)
LCMS-2010EV (Shimadzu)
LCMS-IT-TOF (Shimadzu)
Agilent 6150 (Agilent)
LCQ Deca XP (Thermo Fisher Scientific)

As the iodination method, ESI (Electrospray Ionization) method or a dualionization method of ESI and APCI (Atmospheric Pressure Chemical Ionization) method was used.

The powder X-ray diffraction measurement was carried out by using "MiniFlex600" (radiation source: Cu, wavelength: 1.54 (10⁻¹⁰ m)) manufactured by Rigaku Corporation and "PertPRO" (radiation source: Cu, wavelength: 1.54 (10⁻¹⁰ m)) manufactured by PANalytical were used.

The names of compounds were named by using ACD/Name ver. 12.01 (trade name), etc.

### Example 1

### Method for producing the compound (3) (No. 1)

### (1) (5-Cyclopropyl-6-methoxypyridin-2-yl)[4-(1,1-difluoroethyl)phenyl]methanone

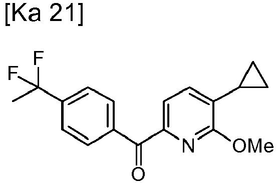

A 1.6M solution of n-butyl lithium in hexane (127 mL) was dropwise added to a solution of 6-bromo-3-cyclopropyl-2-methoxypyridine (41.5 g) in tetrahydrofuran (273 mL) over 50 minutes at -78°C in a nitrogen atmosphere, followed by stirring at -78°C for 1 hour. Then, at -78°C as it was, a solution of 4-(1,1-difluoroethyl)benzonitrile (24.3 g) in tetrahydrofuran (137 mL) was dropwise added to the reaction solution over 75 minutes, followed by stirring for 1 hour. The reaction solution was warmed to 0°C, and then 1 M hydrochloric acid (437 mL), tetrahydrofuran (365 mL) and 1 M hydrochloric acid (146 mL) were dropwise added in this order.

The reaction solution was separated into an organic layer and an aqueous layer, and then the aqueous layer was extracted with ethyl acetate (1,000 mL). The mixed organic layer was dried over anhydrous magnesium sulfate, and the drying agent was filtered off, and then the solvent was distilled off under reduced pressure. The resulting residue was purified by silica gel column chromatography (hexane/ethyl acetate = 100/0 → 19/1, V/V) to obtain the title compound (34.0 g, yield 74%) as a colorless oil.

¹H NMR (300 MHz, CDCl₃) δ ppm 0.72-0.81 (m, 2H), 1.00-1.10 (m, 2H), 1.96 (t, J=18.2 Hz, 3H), 2.10-2.25 (m, 1H), 3.95 (s, 3H), 7.24 (d, J=6.9 Hz, 1H), 7.59 (d, J=9.0 Hz, 2H), 7.67 (d, J=7.8 Hz, 1 H), 8.21 (d, J=8.6 Hz, 2H). MS (+): 318 [M+H]⁺

### (2) Compound (3): 3-cyclopropyl-6-[4-(1,1-difluoroethyl)benzoyl]pyridin-2(1H)-one

Trimethylsilyl chloride (104.36 g) was dropwise added to a solution of (5-cyclopropyl-6-methoxypyridin-2-yl)[4-(1,1-difluoroethyl)phenyl]methanone (76.31 g) and potassium iodide (146.97 g) in acetonitrile (656.07 g) over 5 minutes at 23 to 24°C in a nitrogen atmosphere, followed by heating to 64°C over 3 hours and 32 minutes and stirring at 63 to 64°C for 5 hours and 14 minutes. The mixture was cooled to room temperature and stirred for 14 hours, and then stirred for 1 hour and 30 minutes while heating to 64°C. After cooling, an aqueous solution prepared by mixing sodium carbonate (50.97 g) and water (201.34 g) was dropwise added thereto over 10 minutes at 17 to 30°C, followed by adding water (102.93 g) and distilling the solvent off under reduced pressure so as to be 669.38 g. Ethyl acetate (760.66 g) was added to the resulting residue and mixed, followed by liquid-liquid extraction into an organic layer and an aqueous layer. A 10% sodium thiosulfate aqueous solution (349.82 g) was added to the organic layer and mixed, followed by liquid-liquid extraction into an organic layer and an aqueous layer. A 10% sodium thiosulfate aqueous solution (350.40 g) was added to the organic layer and mixed, followed by liquid-liquid extraction into an organic layer and an aqueous layer. The organic layer was distilled to remove the solvent under reduced pressure so as to be 116.9 g, followed by adding ethyl acetate (1,496.40 g) and heating to 33°C. A 10% sodium thiosulfate aqueous solution (353.05 g) and saturated saline solution (107.24 g) were added thereto and mixed, followed by liquid-liquid extraction into an organic layer and an aqueous layer.

The organic layer was distilled to remove the solvent under reduced pressure so as to be 79.79 g. Then, ethyl acetate (383.57 g) was added to the organic layer, and the mixture was heated to 40°C. Then, normal heptane (385.13 g) was dropwise added to the mixture at 38 to 41 °C over 11 minutes, followed by cooling to 15°C over 42 minutes and stirring for 15 minutes to obtain a suspension. The resulting solid was filtered and washed with a mixed solution of cooled ethyl acetate (77.09 g) and normal heptane (76.26 g), followed by drying under reduced pressure at 50°C for 3 hours to obtain the title compound (67.95 g, yield 63.2%) as a yellow solid.

¹H NMR (300 MHz, CDCl₃) δ ppm 0.78-0.83 (m, 2H), 1.08-1.14 (m, 2H), 1.96 (t, J=18.2 Hz, 3H), 2.25-2.35 (m, 1 H), 6.68 (d, J=6.8 Hz,1 H), 6.91 (d, J=7.2 Hz, 1 H), 7.65 (d, J=8.2 Hz, 2H), 7.80 (d, J=8.2 Hz, 2H), 9.62 (s,1 H).
MS (+): 304 [M+H]⁺

### Example 2

### Method for producing the compound (3) (No. 2)

### (1) (5-Cyclopropylpyridin-2-yl)[4-(1,1-difluoroethyl)phenyl]methanone

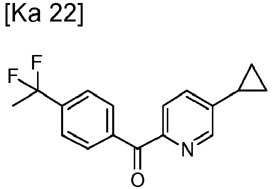

A 1.6M solution of n-butyl lithium in hexane (13.9 mL) was dropwise added to a solution of 1-bromo-4-(1,1-difluoroethyl)benzene (5.54 g) in tetrahydrofuran (5.41 g) over 24 minutes at -70°C or below in a nitrogen atmosphere, followed by washing with tetrahydrofuran (2.71 g). After stirring at -70°C or below for 1 hour, a solution of 5-cyclopropyl picolinonitrile (2.71 g) in tetrahydrofuran (4.20 g) was dropwise added over 15 minutes, followed by washing with tetrahydrofuran (2.74 g). After stirring for 4 hours and 19 minutes, the mixture was warmed to room temperature. Concentrated hydrochloric acid (4.48 g) and water (5.43 g) were added, followed by stirring for 8 minutes, and then the reaction solution was separated into an organic layer and an aqueous layer. Ethyl acetate (11.08 g) was added to the obtained aqueous layer, followed by stirring for 10 minutes, and then the mixture was subjected to liquid-liquid extraction into an organic layer and an aqueous layer. The obtained organic layers were mixed, and an aqueous solution prepared by mixing potassium hydrogen carbonate (1.35 g) and water (8.43 g) was added thereto, followed by stirring for 6 minutes and liquid-liquid extraction into an organic layer and an aqueous layer. An aqueous solution prepared by mixing sodium chloride (0.55 g) and water (8.43 g) was added to the obtained organic layer, followed by stirring for 5 minutes and liquid-liquid extraction into an organic layer and an aqueous layer. The obtained organic layer was subjected to distillation under reduced pressure to remove the solvent. The resulting residue was purified by silica gel column chromatography (hexane/ethyl acetate = 19/1, V/V) to obtain an oily substance (5.87 g).

Hexane (10.19 g) and ethyl acetate (1.30 g) were added to the obtained oily substance, and the mixture was heated to 50°C and then cooled. Then, ethyl acetate (0.51 g) was added to the mixture at 36°C. Then, the mixture was cooled with ice, followed by stirring for 4 minutes so as to be a suspension. The obtained solid was filtered and washed with a mixed solution of ethyl acetate (0.75 g) and hexane (4.95 g), followed by drying under reduced pressure at 40°C to obtain the title compound (3.98 g, yield 72.1%) as a white solid.

¹H NMR (300 MHz, CDCl₃) δ ppm 0.80-0.94 (m, 2H), 1.12-1.24 (m, 2H), 1.84-2.07 (m, 4H), 7.46 (dd, J=2.4, 8.2 Hz, 1 H), 7.60 (d, J=8.6 Hz, 2H), 8.00 (d, J=8.2 Hz, 1 H), 8.10 (d, J=8.2 Hz, 2H), 8.49 (d, J=2.0 Hz, 1 H).
MS (+): 288 [M+H]⁺

### (2) 5-Cyclopropyl-2-[4-(1,1-difluoroethyl)benzoyl]pyridin-1-oxide

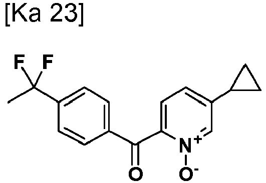

A m-chloroperoxybenzoic acid (30% water, 34.32 g) was added to a solution of (5-cyclopropylpyridin-2-yl)[4-(1,1-difluoroethyl)phenyl]methanone (20.00 g) in chloroform (100.00 g) in a nitrogen atmosphere, followed by washing with chloroform (10.02 g), and then stirring for 4 hours at 25°C. A chloroform, an aqueous solution prepared by mixing sodium thiosulfate (14.31 g) and water (60.01 g) and a 5% sodium hydrogen carbonate aqueous solution (60.01 g) were added to the reaction solution and mixed, followed by liquid-liquid extraction into an organic layer and an aqueous layer. A 5% sodium hydrogen carbonate aqueous solution (60.00 g) was added to the organic layer and mixed, followed by liquid-liquid extraction into an organic layer and an aqueous layer. A 5% sodium hydrogen carbonate aqueous solution (120.01 g) was added to the organic layer and mixed, followed by liquid-liquid extraction into an organic layer and an aqueous layer. A 5% sodium hydrogen carbonate aqueous solution (120.00 g) was added to the organic layer and mixed, followed by liquid-liquid extraction into an organic layer and an aqueous layer. Water (60.02 g) was added to the organic layer and mixed, followed by liquid-liquid extraction into an organic layer and an aqueous layer. The obtained organic layer was distilled under reduced pressure to remove the solvent, and a pale yellow solid (25.33 g) was obtained. The obtained solid was purified by silica gel column chromatography (hexane/ethyl acetate = 1/1, V/V) to obtain the title compound (18.05 g, yield 85.5%) as a pale yellow solid.

¹H NMR (300 MHz, CDCl₃) δ ppm 0.81-0.92 (m, 2H), 1.14-1.28 (m, 2H), 1.91 (t, J=18.2 Hz, 3H), 1.87-2.04 (m, 1 H), 7.07 (dd, J=1.7, 8.3 Hz, 1 H), 7.34 (d, J=8.3 Hz, 1 H), 7.59 (d, J=8.3 Hz, 2H), 7.86 (d, J=8.3 Hz, 2H), 7.99 (d, J=1.4 Hz, 1 H). MS (+): 304[M+H]⁺

### (3) Compound (3): 3-cyclopropyl-6-[4-(1,1-difluoroethyl)benzoyl]pyridin-2(1H)-one

Anhydrous trifluoroacetic acid (59.82 g) was added to a solution of 5-cyclopropyl-2-[4-(1,1-difluoroethyl)benzoyl]pyridin-1-oxide (12.00 g) in 2-methyltetrahydrofuran (120.02 g) in a nitrogen atmosphere, followed by stirring for 7 hours at 23 to 26°C. An aqueous solution prepared by mixing sodium hydroxide (22.15 g) and water (36.00 g) was dropwise added to the reaction solution and then mixed with an aqueous solution prepared by mixing potassium hydrogen carbonate (11.88 g) and water (108.00 g), and chloroform (72.08 g) was added thereto and mixed, followed by liquid-liquid extraction into an organic layer and an aqueous layer. Chloroform (60.00 g) was added to the obtained aqueous layer, followed by stirring and liquid-liquid extraction into an organic layer and an aqueous layer. The obtained organic layers were mixed, and water (60.00 g) was added thereto, followed by stirring and liquid-liquid extraction into an organic layer and an aqueous layer. The obtained organic layer was distilled under reduced pressure to remove the solvent, followed by drying under reduced pressure at room temperature to obtain a pale yellow solid (11.77 g).

Ethyl acetate (47.08 g) was added to the obtained solid, and the mixture was stirred in the state of suspension for 30 minutes at 50 to 51 °C. The mixture was cooled and stirred for 30 minutes at 1°C. The obtained solid was filtered, washed with ethyl acetate (17.67 g) and dried under reduced pressure for 2 hours at 50°C to obtain the title compound (9.07 g, yield 75.6%) as a pale yellow solid.

MS (+): 304 [M+H]⁺

### Example 3

### Method for producing the compound (3) (No. 3)

### (1) 5-Cyclopropyl-6-methoxypicolinonitrile

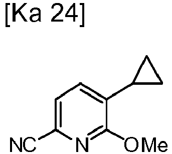

Tris(2,4-pentanedionate) iron(III) (0.05 g) was added to a solution of 5-chloro-6-methoxypicolinonitrile (0.50 g) in tetrahydrofuran (2.51 g) and N-methylpyrrolidone (2.50 g) at 6 to 7°C in a nitrogen atmosphere, followed by adding a 0.7 M solution of cyclopropyl magnesium bromide in tetrahydrofuran (5.93 mL) and stirring for one hour and 15 minutes at 4 to 7°C. Water (5.00 g) and ethyl acetate (5.00 g) were added to the reaction solution and mixed, followed by liquid-liquid extraction into an organic layer and an aqueous layer. Ethyl acetate (5.02 g) was added to the obtained aqueous layer and mixed, followed by liquid-liquid extraction into an organic layer and an aqueous layer. Ethyl acetate (5.01 g) was added to the obtained aqueous layer and mixed, followed by liquid-liquid extraction into an organic layer and an aqueous layer. The obtained organic layers were mixed, and saturated saline solution (5.00 g) was added thereto and mixed, followed by liquid-liquid extraction into an organic layer and an aqueous layer. The obtained organic layer was dried over anhydrous magnesium sulfate, and the drying agent was filtered off, followed by distillation under reduced pressure to remove the solvent. The resulting residue was purified by silica gel column chromatography (hexane and ethyl acetate) to obtain the title compound (0.29 g, yield 55.8%) as a pale yellow solid.

¹H NMR (600 MHz, CDCl₃) δ ppm 0.69-0.75 (m, 2H), 1.02-1.09 (m, 2H), 2.10-2.16 (m, 1 H), 4.00 (s, 3H), 7.10 (d, J=7.4 Hz, 1 H), 7.20 (d, J=7.4 Hz, 1 H). MS (+): 175 [M+H]⁺

### (2) (5-Cyclopropyl-6-methoxypyridin-2-yl)[4-(1,1-difluoroethyl)phenyl]methanone

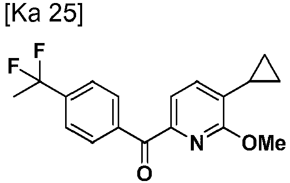

A 1.6 M solution of n-butyl lithium in hexane (0.4 mL) was dropwise added to a solution of 1-bromo-4-(1,1-difluoroethyl)benzene (0.17 g) in tetrahydrofuran (0.61 g) at-60°C or below, followed by stirring for 1 hour, and a solution of 5-cyclopropyl-6-methoxypicolinonitrile (0.10 g) in tetrahydrofuran (0.30 g) was dropwise added over 4 minutes. After stirring for 2 hours and 2 minutes, the mixture was warmed to room temperature. A 5% hydrochloric acid aqueous solution (0.93 g) was added thereto, followed by stirring, and then water and ethyl acetate were added thereto and mixed, followed by liquid-liquid extraction into an organic layer and an aqueous layer. Ethyl acetate (4 mL) was added to the obtained aqueous layer and mixed, followed by liquid-liquid extraction into an organic layer and an aqueous layer. Both the organic layers were mixed, and a 5% sodium hydrogen carbonate aqueous solution was added thereto and mixed, followed by liquid-liquid extraction into an organic layer and an aqueous layer. Ethyl acetate (2 mL) was added to the obtained aqueous layer and mixed, followed by liquid-liquid extraction into an organic layer and an aqueous layer. Both the organic layers were mixed, and water was added thereto and mixed, followed by liquid-liquid extraction into an organic layer and an aqueous layer. Ethyl acetate (2 mL) was added to the obtained aqueous layer and mixed, followed by liquid-liquid extraction into an organic layer and an aqueous layer. Both the organic layers were mixed, and sodium sulfate was added thereto and dried. Then, the mixture was filtered to remove the drying agent, followed by distillation under reduced pressure to remove the solvent. The resulting residue was purified by silica gel column chromatography (hexane/ethyl acetate = 49/1, V/V) to obtain the title compound (0.14 g, yield 75.1%) as a pale yellow oil.

MS (+): 318 [M+H]⁺

### (3) Compound (3): 3-cyclopropyl-6-[4-(1,1-difluoroethyl)benzoyl]pyridin-2(1H)-one

A compound was prepared by the same method as in Example 1-(2).

### Reference Example 1 (the production method described in Patent Document 1 was applied)

Preparation of (5R)-5-{(Z)-2-(5-cyclopropyl-6-methoxypyridin-2-yl)-2-[4-(1,1-difluoroethyl)phenyl]ethenyl}pyrrolidin-2-one (Reference Example 1 a) and (5R)-5-{(E)-2-(5-cyclopropyl-6-methoxypyridin-2-yl)-2-[4-(1,1-difluoroethyl)phenyl]ethenyl}pyrrolidin-2-one (Reference Example 1 b)

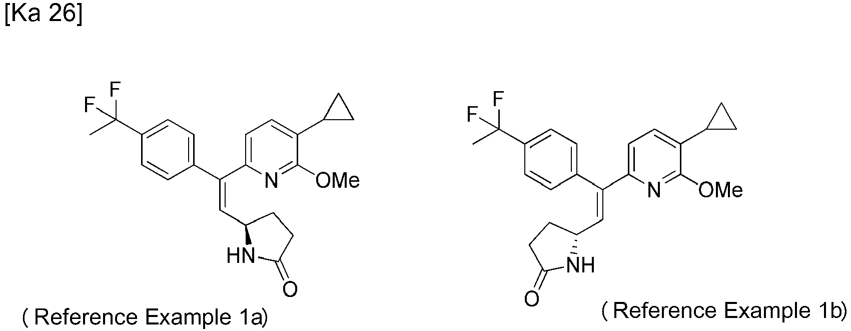

A 1 M solution of lithium bis(trimethylsilyl)amide in tetrahydrofuran (22.0 mL) was dropwise added to a solution of (5-cyclopropyl-6-methoxypyridin-2-yl)[4-(1,1-difluoroethyl)phenyl]methanone (1.00 g) and (R)-5-[(benzothiazol-2-yl sulfonyl)methyl]pyrrolidin-2-one (1.90 g) in tetrahydrofuran (6.00 g) and 1,3-dimethyl-2-imidazolidinone (4.02 g) over 30 minutes at -17 to -12°C in a nitrogen atmosphere, followed by stirring for 1 hour and 50 minutes at -17 to -10°C. The mixture was warmed to 22°C, and water (10.10 g) was added thereto, followed by stirring for 1 hour and 4 minutes. Then, a 50% sulfuric acid aqueous solution (4.78 g) was added thereto, followed by distillation under reduced pressure to remove the solvent. Ethyl acetate (20.00 g) and water (20.00 g) were added to the resulting residue and mixed, followed by liquid-liquid extraction into an organic layer and an aqueous layer. A saturated sodium hydrogen carbonate aqueous solution (20.00 g) was added to the obtained organic layer and mixed, followed by liquid-liquid extraction into an organic layer and an aqueous layer. A saline solution was added to the obtained organic layer and mixed, followed by liquid-liquid extraction into an organic layer and an aqueous layer. The obtained organic layer was distilled under reduced pressure to remove the solvent, and the resulting residue was purified by silica gel column chromatography (hexane and ethyl acetate) to obtain a mixture (1.10 g) containing the title compound as an brown amorphous substance. Based on calculation of the proportion of the area ratio of the characteristic peaks in ¹H NMR data, the yield of (5R)-5-{(Z)-2-(5-cyclopropyl-6-methoxypyridin-2-yl)-2-[4-(1,1-difluoroethyl)phenyl]ethenyl}pyrrolidin-2-one was 49.3%, and the yield of (5R)-5-{(E)-2-(5-cyclopropyl-6-methoxypyridin-2-yl)-2-[4-(1,1-difluoroethyl)phenyl]ethenyl}pyrrolidin-2-one was 38.0%.

### (5R)-5-{(Z)-2-(5-cyclopropyl-6-methoxypyridin-2-yl)-2-[4-(1,1-difluoroethyl)phenyl]ethenyl}pyrrolidin-2-one (Reference Example 1 a)

¹H NMR (300 MHz, CDCl₃) δ ppm 0.65-0.71 (m, 2H), 0.96-1.02 (m, 2H), 1.86-2.16 (m, 5H), 2.28-2.49 (m, 3H), 4.00 (s, 3H), 4.65-4.72 (m, 1 H), 5.91 (d, J=8.9 Hz, 1 H), 5.96 (br, 1 H), 6.54 (d, J=7.7 Hz, 1 H), 7.05 (d, J=7.0 Hz, 1 H), 7.30 (d, J=8.6 Hz, 2H), 7.44 (d, J=8.6 Hz, 2H).

### MS (+): 399 [M+H]⁺

### (5R)-5-{(E)-2-(5-cyclopropyl-6-methoxypyridin-2-yl)-2-[4-(1,1-difluoroethyl)phenyl]ethenyl}pyrrolidin-2-one (Reference Example 1 b)

¹H NMR (600 MHz, CDCl₃) δ ppm 0.56-0.64 (m, 2H), 0.90-0.97 (m, 2H), 1.93-2.09 (m, 5H), 2.20-2.34 (m, 2H), 2.37-2.45 (m, 1 H), 4.04 (s, 3H), 4.07-4.16 (m, 1 H), 5.73-5.75 (br. s., 1 H), 6.23 (d, J=7.4 Hz, 1 H), 6.90 (d, J=9.9 Hz, 1 H), 6.92 (d, J=7.8 Hz, 1 H), 7.24 (d, J=8.3 Hz, 2H), 7.57 (d, J=7.8 Hz, 2H).

### MS (+): 399 [M+H]⁺

In Examples 4 and 5, the reaction yield was calculated by a quantitative analysis method using HPLC, (R, Z)-3-cyclopropyl-6-{1-[4-(1,1-difluoroethyl)phenyl]-2-[5-oxopyrrolidin-2-yl]vinyl}pyridin-2(1H)-one and (R, E)-3-cyclopropyl-6-{1-[4-(1,1-difluoroethyl)phenyl]-2-[5-oxopyrrolidin-2-yl]vinyl}pyridin-2(1H)-one which were purified by silica gel column chromatography or the like as standard substances and phthalic acid di(2-ethyl hexyl) ester as an internal standard substance.
Column: L-Column ODS (3.0 x 150 mm, 3 µm) (manufactured by Chemical Evaluation and Research Institute, Japan)
Column oven temperature: 40°C
Elute: Acetonitrile-0.01 M ammonium acetate aqueous solution, 22:78 (0-15 min), 22:78-40:60 (15-20 min), 40:60 (20-30 min), 40:60-95:5 (30-40 min), 95:5 (40-55 min), V/V
Elute rate: 0.4 mL/min
Detection wavelength: 240 nm

### Example 4

Preparation of (R, Z)-3-cyclopropyl-6-{1-[4-(1,1-difluoroethyl)phenyl]-2-[5-oxopyrrolidin-2-yl]vinyl}pyridin-2(1 H)-one (Example 4a) and (R, E)-3-cyclopropyl-6-{1-[4-(1,1-difluoroethyl)phenyl]-2-[5-oxopyrrolidin-2-yl]vinyl}pyridin-2(1H)-one (Example 4b)

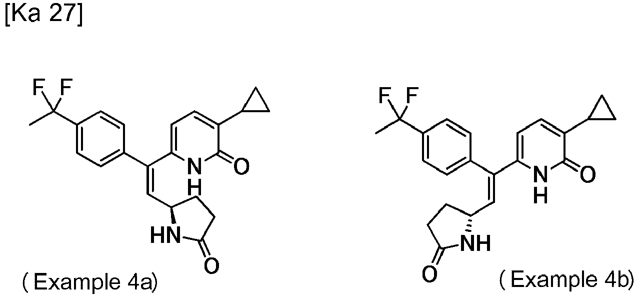

A 1 M solution of lithium bis(trimethylsilyl)amide in tetrahydrofuran (23.0 mL) was dropwise added to a solution of 3-cyclopropyl-6-[4-(1,1-difluoroethyl)benzoyl]pyridin-2(1 H)-one (1.00 g) and (5R)-5-[(1,3-benzothiazol-2-yl-sulfonyl)methyl]pyrrolidin-2-one (1.95 g) in tetrahydrofuran (6.03 g) and 1,3-dimethyl-2-imidazolidinone (4.02 g) over 30 minutes at -12 to -10°C in a nitrogen atmosphere, followed by stirring at -11 to -10°C for 1 hour and 30 minutes. The reaction solution was warmed to room temperature and subjected to quantitative determination. As a result, the quantitative yield of (R, Z)-3-cyclopropyl-6-{1-[4-(1,1-difluoroethyl)phenyl]-2-[5-oxopyrrolidin-2-yl]vinyl}pyridin-2(1H)-one as the main component (desired compound) was 77.7%, and the quantitative yield of (R, E)-3-cyclopropyl-6-{1-[4-(1,1-difluoroethyl)phenyl]-2-[5-oxopyrrolidin-2-yl]vinyl}pyridin-2(1 H)-one as a by-product was 9.2%.

### (R, Z)-3-cyclopropyl-6-{1-[4-(1,1-difluoroethyl)phenyl]-2-[5-oxopyrrolidin-2-yl]vinyl}pyridin-2(1H)-one (Example 4a)

¹H NMR (300 MHz, CDCl₃) δ ppm 0.61-0.71 (m, 2H), 0.90-1.03 (m, 1H), 1.14-1.30 (m, 1 H), 1.92 (t, J=18.4 Hz, 3H), 1.87-2.00 (m, 1 H), 2.09-2.18 (m, 1 H), 2.28-2.50 (m, 3H), 4.33-4.41 (m, 1H), 6.05 (d, J=7.4 Hz, 1H), 6.16 (d, J=10.2 Hz, 1H), 7.03 (d, J=7.0 Hz, 1H), 7.35 (d, J=8.6 Hz, 2H), 7.46 (d, J=8.6 Hz, 1H), 7.81 (br, 1H), 13.17 (br, 1H). MS (+): 385 [M+H]⁺

### (R, E)-3-cyclopropyl-6-{1-[4-(1,1-difluoroethyl)phenyl]-2-[5-oxopyrrolidin-2-yl]vinyl}pyridin-2(1H)-one (Example 4b)

¹H NMR (300 MHz, CDCl₃) δ ppm 0.50-0.65 (m, 2H), 0.90-1.03 (m, 2H), 1.91-2.46 (m, 8H), 4.07-4.15 (m, 1 H), 5.63 (d, J=7.4 Hz, 1 H), 6.63 (d, J=9.2 Hz, 1 H), 6.80 (dd, J=0.7, 7.4 Hz, 1H), 7.15 (s, 1H), 7.25 (d, J=9.2 Hz, 2H), 7.56 (d, J=8.5 Hz, 2H), 12.46 (br, 1 H).
MS (+): 385 [M+H]⁺

### Example 5

Preparation of (R, Z)-3-cyclopropyl-6-{1-[4-(1,1-difluoroethyl)phenyl]-2-[5-oxopyrrolidin-2-yl]vinyl}pyridin-2(1H)-one (Example 5a) and (R, E)-3-cyclopropyl-6-{1-[4-(1,1-difluoroethyl)phenyl]-2-[5-oxopyrrolidin-2-yl]vinyl}pyridin-2(1H)-one (Example 5b)

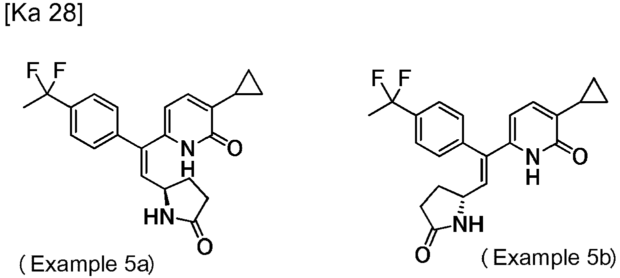

A 1 M solution of lithium bis(trimethylsilyl)amide in tetrahydrofuran (23.0 mL) was dropwise added to a solution of 3-cyclopropyl-6-[4-(1,1-difluoroethyl)benzoyl]pyridin-2(1 H)-one (1.00 g) and (5R)-5-[(1,3-benzothiazol-2-ylsulfonyl)methyl]pyrrolidin-2-one (1.95 g) in tetrahydrofuran (10.00 g) over 30 minutes at -11 to -9°C under a nitrogen atmosphere, followed by stirring at -11 to -9°C for 3 hours and 35 minutes. The reaction solution was warmed to room temperature and subjected to quantitative determination. As a result, the quantitative yield of (R, Z)-3-cyclopropyl-6-{1-[4-(1,1-difluoroethyl)phenyl]-2-[5-oxopyrrolidin-2-yl]vinyl}pyridin-2(1H)-one as the main component (desired compound) was 66.2%, and the quantitative yield of (R, E)-3-cyclopropyl-6-{1-[4-(1,1-difluoroethyl)phenyl]-2-[5-oxopyrrolidin-2-yl]vinyl}pyridin-2(1H)-one as a by-product was 11.5%.

### (R, Z)-3-cyclopropyl-6-{1-[4-(1,1-difluoroethyl)phenyl]-2-[5-oxopyrrolidin-2-yl]vinyl}pyridin-2(1H)-one (Example 5a)

MS (+): 385 [M+H]⁺

### (R, E)-3-cyclopropyl-6-{1-[4-(1,1-difluoroethyl)phenyl]-2-[5-oxopyrrolidin-2-yl]vinyl}pyridin-2(1H)-one (Example 5b)

MS (+): 385 [M+H]⁺

Results of Reference Example 1, Example 4 and Example 5 are shown in Table 1 below. In the table, the additive is 1,3-dimethyl-2-imidazolidinone.

**[Table 1]**

| | Substrate (ketone) | Additive | (E) (Yield%) | (Z) (Yield%) | (E) / (Z) (Ratio) |
|---|---|---|---|---|---|
| Reference Example 1 | 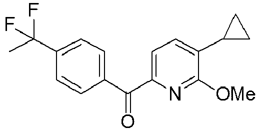 | Added | 38.0 | 49.3 | 1/1.3 |
| Example 4 | 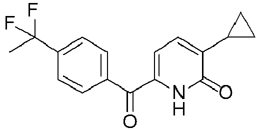 | Added | 9.2 | 77.7 | 1/8.4 |
| Example 5 | 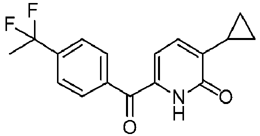 | Not added | 11.5 | 66.2 | 1/5.8 |

Comparing Reference Example 1 with Example 4, it is evident that the selectivity and the yield of the compound in the form of Z isomer improved in Example 4. Further, comparing Example 4 with Example 5, it is evident that by adding 1,3-dimethyl-2-imidazolidinone, the selectivity and the yield of the compound in the form of Z isomer more improved.

### Example 6

Preparation of sodium (R, Z)-3-cyclopropyl-6-{1-[4-(1,1-difluoroethyl)phenyl]-2-[5-oxopyrrolidin-2-yl]vinyl}pyridin-2-olate

A solution of 3-cyclopropyl-6-[4-(1,1-difluoroethyl)benzoyl]pyridin-2(1H)-one (40.00 g) and (5R)-5-[(1,3-benzothiazol-2-yl sulfonyl)methyl]pyrrolidin-2-one (78.20 g) in tetrahydrofuran (160.12 g) and 1,3-dimethyl-2-imidazolidinone (160.10 g) and a 1 M solution of lithium bis(trimethylsilyl)amide in tetrahydrofuran solution (930 mL) were simultaneously dropwise added to tetrahydrofuran (80.1 g) at -15 to -13°C under a nitrogen atmosphere over 1 hour and 50 minutes, followed by washing with tetrahydrofuran (80.06 g). The mixture was stirred at -17 to 15°C for 1 hour and warmed to 12°C, followed by adding water (400.19 g) thereto and stirring at 13 to 15°C for 2 hours and 5 minutes. Then, a 50% sulfuric acid aqueous solution (199.30 g) was added, followed by distillation under reduced pressure to remove the solvent so as to be 857.01 g. Ethyl acetate (400.81 g) and water (600.22 g) were added to the resulting residue, followed by stirring and liquid-liquid extraction into an organic layer and an aqueous layer. An aqueous solution prepared by mixing sodium carbonate (30.02 g) and water (570.00 g) were added to the obtained organic layer and mixed, followed by liquid-liquid extraction into an organic layer and an aqueous layer. An aqueous solution prepared by mixing sodium chloride (30.00 g) and water (570.01 g) were added to the obtained organic layer and mixed, followed by liquid-liquid extraction into an organic layer and an aqueous layer. The obtained organic layer was distilled under reduced pressure to remove the solvent, followed by adding ethyl acetate so as to be 800.12 g. While stirring, the obtained solution was cooled to -1°C, and then a methanol solution (43.26 g) of 28% sodium methoxide was dropwise added thereto over 1 hour, followed by stirring at -4 to -2°C for 1 hour to obtain a suspension. The obtained solid was filtered and washed with ethyl acetate (160.00 g), followed by drying under reduced pressure at 60°C to obtain the title compound (34.36 g, yield 67.8%) as a white solid.

The solid obtained in Example 6 was subjected to powder X-ray diffraction measurement, as a result the following characteristic peaks were measured.

### (Characteristic peaks)

2θ = 6.8, 7.5, 8.6, 11.4, 13.1, 13.5, 16.0, 17.3, 19.5, 20.6, 23.5

Further, the powder X-ray diffraction pattern obtained by the measurement is shown in Fig. 1.

### Example 7

Preparation of 3-cyclopropyl-6-{(1 R)-1-[4-(1,1-difluoroethyl)phenyl]-2-[(2R)-5-oxopyrrolidin-2-yl]ethyl}pyridin-2(1 H)-one

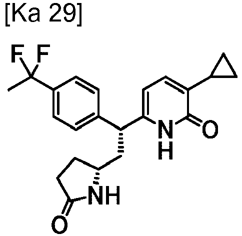

### (1) (R, Z)-tert-butyl-6-{2-[1-(tert-butoxycarbonyl)-5-oxopyrrolidin-2-yl]-1-[4-(1,1-difluoroethyl)phenyl]vinyl}-3-cyclopropyl-2-oxopyridin-1(2H)-carboxylate

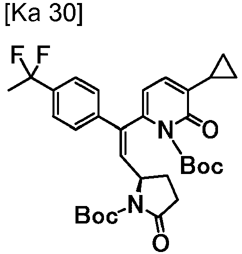

A 10% ammonium chloride aqueous solution (100 g) and ethyl acetate (100 g) were added to sodium (R, Z)-3-cyclopropyl-6-{1-[4-(1,1-difluoroethyl)phenyl]-2-[5-oxopyrrolidin-2-yl]vinyl}pyridin-2-olate (10.00 g), followed by stirring and then liquid-liquid extraction into an organic layer and an aqueous layer.

Water (50 g) was added to the organic layer, followed by stirring and then liquid-liquid extraction into an organic layer and an aqueous layer. The obtained organic layer was distilled under reduced pressure to remove the solvent, and ethyl acetate (50 g) was added to the resulting residue, followed by distillation under reduced pressure to remove the solvent. Acetonitrile (50 g) was added to the resulting residue, followed by distillation under reduced pressure to remove the solvent. The resulting residue was mixed with acetonitrile (70 g), triethylamine (8.73 g) and N,N-dimethyl-4-aminopyridine (0.609 g), followed by dropwise adding a solution prepared by mixing di-tert-butyl dicarbonate (16.12 g) and acetonitrile (30 g) thereto over 6 minutes at 24°C. The mixture was heated to 40°C and stirred for 1 hour and 35 minutes, followed by distillation under reduced pressure to remove the solvent. A 10% ammonium chloride aqueous solution (100 g) and ethyl acetate (100 g) were added to the resulting residue and mixed, followed by liquid-liquid extraction into an organic layer and an aqueous layer. Water (50 g) was added to the organic layer and mixed, followed by liquid-liquid extraction into an organic layer and an aqueous layer. Ethyl acetate (50 g) was added to the obtained aqueous layer and mixed, followed by liquid-liquid extraction into an organic layer and an aqueous layer. The obtained organic layers were mixed, followed by distillation under reduced pressure to remove the solvent. Toluene (30 g) was added to the resulting residue, followed by distillation under reduced pressure to remove the solvent. The resulting residue was purified by silica gel column chromatography (hexane/ethyl acetate = 77/23 → 73/27, V/V) to obtain the title compound (12.10 g, yield 84.1 %) as a pale yellow amorphous substance.

¹H NMR (300 MHz, CDCl₃) δ ppm 0.70-0.75 (m, 2H), 1.01-1.07 (m, 2H), 1.40 (s, 9H), 1.58 (s, 9H), 1.93 (t, J=18.0 Hz, 3H), 1.96-2.08 (m, 2H), 2.44-2.62 (m, 3H), 5.06-5.14 (m, 1 H), 6.04 (d, J=9.0 Hz, 1 H), 6.91 (d, J=7.8 Hz, 1 H), 7.26 (d, J=7.3 Hz, 1 H), 7.28 (d, J=7.0 Hz, 2H), 7.46 (d, J=8.2 Hz, 2H).
MS (+): 585 [M+H]⁺

### (2) tert-butyl-6-{(R)-2-[(R)-1-(tert-butoxycarbonyl)-5-oxopyrrolidin-2-yl]-1-[4-(1,1-difluoroethyl)phenyl]ethyl}-3-cyclopropyl-2-oxopyridine-1(2H)-carboxylate

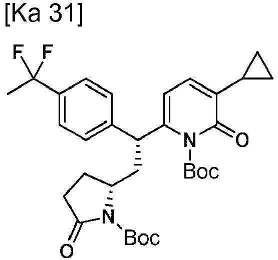

(R, Z)-tert-butyl-6-{2-[1-(tert-butoxycarbonyl)-5-oxopyrrolidin-2-yl]-1-[4-(1,1-difluoroethyl)phenyl]vinyl}-3-cyclopropyl-2-oxopyridine-1(2H)-carboxylate (7.00 g) was mixed with ethyl acetate (35 g) and 5%palladium-activated carbon (1.40 g, wetted with 49.81 % water), followed by strirring at 22 to 23°C for 3 hours under a hydrogen atmosphere. The reaction solution was filtered, the resulting residue was washed with ethyl acetate (70 g), and the filtrate was added to the residue, followed by distillation to remove the solvent. Then, toluene (20 g) was added thereto, followed by distillation under reduced pressure to remove the solvent.

The resulting residue was purified by silica gel column chromatography (hexane/ethyl acetate = 71/29 → 65/35, V/V) to obtain the title compound (5.55 g, yield 79.1 %) as a white amorphous substance.

¹H NMR (300 MHz, CDCl₃) δ ppm 0.61-0.66 (m, 2H), 0.91-0.98 (m, 2H), 1.47-1.65 (m, 1 H), 1.48 (s, 9H), 1.56 (s, 9H), 1.69-1.77 (m, 1 H), 1.86-2.17 (m, 2H), 1.88 (t, J=18.2 Hz, 3H), 2.30-2.54 (m, 2H), 2.79-2.87 (m, 1H), 4.07-4.16 (m, 2H), 7.00 (d, J=7.8 Hz, 1 H), 7.24 (d, J=7.0 Hz, 1 H), 7.36 (d, J=8.6 Hz, 2H), 7.42 (d, J=8.6 Hz, 2H). MS (+): 587 [M+H]⁺

### (3) 3-Cyclopropyl-6-{(1R)-1-[4-(1,1-difluoroethyl)phenyl]-2-[(2R)-5-oxopyrrolidin-2-yl]ethyl}pyridin-2(1H)-one

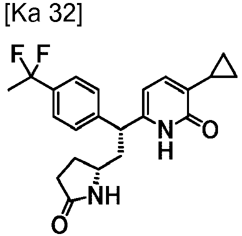

35% hydrochloric acid (30.78 g) was dropwise added to an ethyl acetate solution (376.48 g) containing tert-butyl 6-{(R)-2-[(R)-1-(tert-butoxycarbonyl)-5-oxopyrrolidin-2-yl]-1-[4-(1,1-difluoroethyl)phenyl]ethyl}-3-cyclopropyl-2-oxopyrrolidine-1 (2H)-carboxylate (39.95 g) at 32 to 34°C over 30 minutes, followed by stirring at 32 to 34°C for 4 hours. An aqueous solution prepared by mixing sodium hydroxide (38.40 g) and water (150.10 g) was dropwise added thereto, followed by stirring at 33 to 35°C and then liquid-liquid extraction into an organic layer and an aqueous layer. An aqueous solution prepared by mixing potassium hydrogen carbonate (29.88 g) and water (150.00 g) was added to the obtained organic layer and mixed, followed by liquid-liquid extraction into an organic layer and an aqueous layer. The obtained organic layer was distilled under reduced pressure to remove the solvent, and then ethanol (300.07 g) was added to the resulting residue, followed by distillation under reduced pressure to remove the solvent. Ethanol (300.00 g) was added to the resulting residue, followed by distillation under reduced pressure to remove the solvent.

Ethanol was added to the resulting residue so as to be 210.02 g, followed by stirring at 18 to 20°C for 1 hour to be a suspension, and then the suspension was heated to 56°C over 3 hours and stirred for 1 hour. Then, the suspension was cooled to -2°C and then stirred for 103 hours. The resulting solid was filtered, washed wish ethanol (120.00 g) and dried under reduced pressure at 60°C to obtain the title compound (22.22 g, yield 84.5%) as a white solid.

¹H NMR (300 MHz, CDCl₃) δ ppm 0.56-0.67 (m, 2H), 0.91-0.98 (m, 2H), 1.66-1.78 (m, 1 H), 1.89 (t, J=18.1 Hz, 3H), 2.07-2.42 (m, 6H), 3.44-3.53 (m, 1 H), 4.08 (dd, J=6.0, 9.6 Hz, 1 H), 5.97 (d, J=7.4 Hz, 1 H), 6.91 (d, J=7.0 Hz, 1 H), 7.44 (s, 4H), 7.49 (br, 1 H), 12.36 (br, 1 H).
MS (+): 387 [M+H]⁺

### INDUSTRIAL APPLICABILITY

The present invention is useful, since the 2-pyridone compound which is useful as a pharmaceutical or an intermediate for a pharmaceutical can be produced at a high yield from a 6-benzoyl-2-pyridone compound.

The entire disclosure of Japanese Patent Application No. 2014-099755 filed on May 13, 2014 including specification, claims, drawings and summary is incorporated herein by reference in its entirety.

## Claims

1. A method for producing a 2-pyridone compound represented by the formula (1), which comprises reacting a 6-benzoyl-2-pyridone compound represented by the formula (3) with a sulfone compound represented by the formula (4):

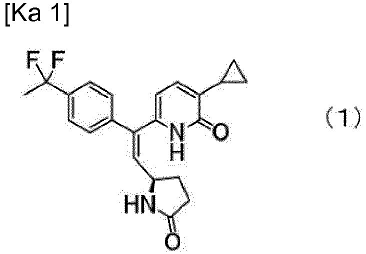

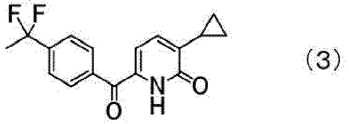

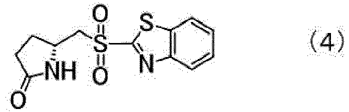

2. The production method according to Claim 1, wherein the reaction is carried out in the presence of a urea derivative.

3. The production method according to Claim 2, wherein the urea derivative is 1,3-dimethyl-2-imidazolidinone.

4. A compound represented by the formula (1):

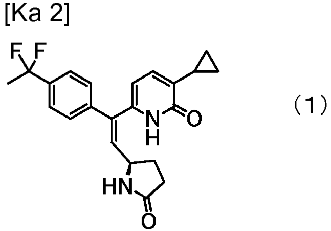

5. A compound represented by the formula (3):

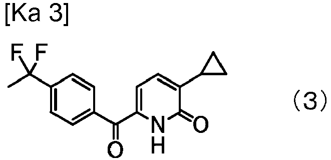

6. A sodium salt of a compound represented by the formula (1):

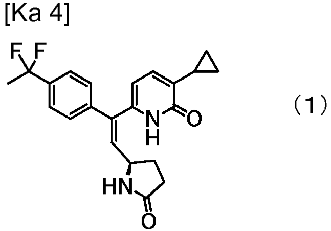

7. A compound represented by the formula (6):

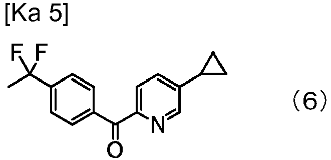

8. A compound represented by the formula (7):

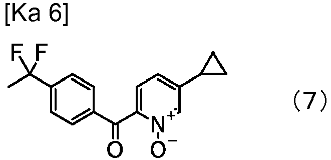

9. A compound represented by the formula (8):

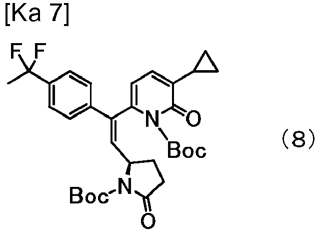

10. A compound represented by the formula (9):

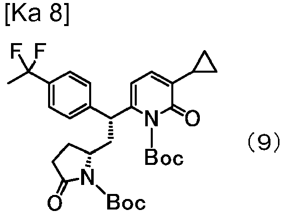

11. A compound represented by the formula (10):
